# EUROPEAN PATENT APPLICATION

(11) **EP 1 281 757 A1**
(43) Date of publication of application: **05.02.2003**
(21) Application number: 01118416.5
(22) Date of filing: 31.07.2001
(51) Int. Cl.: C12N 15/10, C12Q 1/68

(54) **Method for the production of nucleic acids consisting of stochastically combined parts of source nucleic acids**

(71) Applicant: Direvo Biotech AG, 50829 Köln (DE)
(72) Inventor: Koltermann, Andre, Dr. c/o DIREVO BIOTECH AG, 50829 Köln (DE); Kettling, Ulrich, Dr. c/o DIREVO BIOTECH AG, 50829 Köln (DE); Greiner-Stöffele, Thomas, Dr. c/ DIREVO BIOTECH AG, 50829 Köln (DE)
(74) Representative: Helbing, Jörg, Dr. Dipl.-Chem.

(57) **Abstract**

Methods and kit for producing recombinant chimeric polynucleotides comprising: forming heteroduplices with single-stranded, partially heterologous source nucleic acids which contain at least one marker nucleotide, introducing single-stranded nicks at sites of the marker nucleotides, and starting synthesis of single-stranded nucleic acids at the nick sites, the non-nicked strand serving as template.

SEQUENCE LISTING

<110> DIREVO Biotech AG

<120> Method for the Production of Nucleic Acids Consisting
      of Stochastically Combined Parts of Source Nucleic
      Acids

<130> 011743ep/JH/ml

<140>
<141>

<160> 2

<170> PatentIn Ver. 2.1

<210> 1
<211> 22
<212> DNA
<213> Artificial Sequence

<220>
<223> Description of Artificial Sequence: Primer

<400> 1
ccagtcacga cgttgtaaaa cg                                          22


<210> 2
<211> 24
<212> DNA
<213> Artificial Sequence

<220>
<223> Description of Artificial Sequence: Primer

<400> 2
taacaatttc acacaggaaa cagc                                        24

## Description

The present invention relates to a method for the production of nucleic acids consisting of stochastically combined parts of source nucleic acids as well as to a kit for carrying out said method.

In nature, nucleic acids provide the biological information that determines structure and function of proteins and, thereby, controls the entire functionality of living beings, from the simplest bacterial cell to very complex multi-cellular organisms. It has been shown, that proteins can be engineered to have new or altered properties that can be exploited for technical or medical purposes. Such engineering can be done by modifying the nucleic acid sequence coding for the corresponding protein, expressing the protein by means of an expression system, testing the protein properties by a sufficiently powerful screening technique and selecting those that are best performers. Of course, when nucleic acids serve as functional molecules itselves, this procedure can be employed as well. Whenever the procedure is done in an iterative manner, the technique is termed directed evolution by analogy to nature's way to generate new functions and alter existing ones.

The modification of nucleic acids is an intrinsic step in directed evolution. Besides the introduction of punctual mutations, the recombination of sequence parts is a very successful strategy for modifying nucleic acids and for generating diverse libraries that can be subjected to screening and selection procedures afterwards. Sequence parts may be fragments of a genome, gene clusters, genes variants within a gene cluster, parts of genes such as exons, or sequences coding for domains within a protein, but may also be very short nucleic acid fragments down to few or even single nucleobases.

Recombination of parts of nucleic acids is preferably done by homologous recombination. Homologous recombination is the combination of corresponding sequence parts from different source nucleic acids while maintaining orientation and reading frame. Main advantage of homologous recombination is the prevention of background noise of unrelated sequences that accompanies an unspecific recombination.

Experimentally, homologous recombination is preferably done *in vitro* using individual enzymatic functions or defined mixtures or sequences of enzymatic processing steps.

A first *in vitro* method described in WO 95/22625 is PCR-based (see also Stemmer, Nature 370 (1994) 389). Here, overlapping gene fragments are provided and are subsequently assembled into products of original length by a PCR without addition of primers. Thus, the mutual priming of the fragments in each PCR cycle allows for fragments of different origin to be incidentally linked to form a product molecule. Theoretically, recombination events introduced by this method are stochastically distributed over the whole resulting nucleic acid sequence. The number of recombination events per nucleic acid molecule, i.e. the frequency of recombination, and also the average distance between recombination sites is determined by the fragment length. On the other hand, the minimal fragment size is in the order of hundreds of base pairs in order to enable mutual priming at a sufficient rate. The shorter the fragments the lower is the probability of efficient annealing of fragments. Therefore, the number of recombination events per gene is limited and, moreover, the minimal average distance of recombination sites is restricted. No means is provided to control these factors.

Another PCR-based method is described in WO 98/42728 (Shao et al., Nucl. Acids Res. 26 (1998), 681). Here, primers with randomized sequences are used which enable a start of polymerization at random positions within a polynucleotide. Thus, similar to WO 95/22625, short polynucleotide fragments are formed which can recombine with each other by mutual priming. With this method, controlling the frequency and distance of the recombinations is hardly possible. Moreover, unspecific primers lead to a comparatively high inherent error rate which can constitute a problem with sensitive sequence parts and/or long genes.

Another method described in WO 98/42728 uses a modified PCR protocol to provoke a strand exchange during the primer extension step in PCR (Zhao et al., Nat. Biotechnol. 16 (1998), 258). The method consists of priming template sequences with a primer followed by repeated cycles of denaturation and extremely abbreviated annealing and polymerase-catalyzed extension. In each cycle the growing fragments can anneal to different templates based on sequence complementarity and extend further. This is repeated until full-length sequences form. Due to template switching, resulting polynucleotides can contain sequence information from different parental sequences. Accordingly, the recombination frequency is controlled by the number of PCR cycles while the average distance between recombination sites is determined by the actual setting of the polymerization time. Due to technical limitations of provoking fast temperature shifts, the minimal average distance between recombination sites is in the range of hundred nucleobases.

WO 01/34835 describes a method for homologous recombination that is not PCR-based. This method combines the controllability of the recombination frequency with the possibility of regio-selective recombination. The method employs partial exonucleolytic single-strand degradation and template-directed single-strand synthesis of double-stranded heteroduplices that are formed by melting and reannealing of source nucleic acids. Multiple recombinations are achieved by repeating the degradation and re-synthesis steps in an iterative manner. Accordingly, the number of cycles determines the recombination frequency. By controlling the exonucleolytic activity, the method allows for regioselective recombination. Very short distances between recombination sites are practically only achieved when focussing on a certain region in the range of hundred nucleobases in the source nucleic acid molecules. Short average distances over the entire source nucleic acid sequences are difficult to achieve.

Another method for homologous recombination that is not PCR-based is described in WO 01/29211. The method relies on the ordering, trimming and joining of randomly cleaved parental DNA fragments annealed to a transient polynucleotide scaffold. As for WO 95/22625, the minimal length of the generated fragments is limited by the necessity of an efficient annealing to the template. Therefore, the minimal distance between recombination sites is not below several hundred nucleobases.

Thus, the technical problem underlying the present invention is to provide a method for the production of nucleic acids consisting of stochastically combined parts of source nucleic acids. Especially, the technical problem is to provide an *in vitro* homologous recombination method that enables very short average distances between recombination sites in combination with controlling the recombination frequency.

### Summary of the Invention

The technical problem has been solved by providing the embodiments characterized in the claims. The present invention thus provides
(A) a method for the production of polynucleotide molecules with modified properties, comprising the following steps:
   (1) providing a population of source nucleic acid molecules, the individual nucleic acid molecules of said population having homologous and heterologous segments and having at least one marker nucleotide incorporated within its nucleic acid sequence;
   (2) forming double-stranded polynucleotide molecules of the population of source nucleic acid molecules provided according to step (1) comprising double strands with heterologous segments (heteroduplices);
   (3) producing single-stranded breaks at the incorporated marker nucleotides of the double-stranded heteroduplices produced according to step (2); and
   (4) performing template-directed single-strand synthesis, with or without incorporation of marker nucleotides starting from single-stranded breaks produced according to step (3); and
(B) a kit for carrying out the method as defined in (A) above, preferably said kit containing at least one of the following components:
   (i) buffer for production of double-stranded polynucleotides;
   (ii) marker nucleotides for incorporation in the polynucleotide molecules;
   (iii) agents permitting the single-stranded breaks at the incorporated marker nucleotides;
   (iv) buffers for carrying out the incorporation of the marker nucleotides and producing the single-stranded breaks at these sites;
   (v) agents permitting the template-directed polymerization of a polynucleotide strand starting from the single-stranded break; and
   (vi) buffer for carrying out this polymerization reaction.

In the method of embodiment (A) of the invention, in step (1) - if the source nucleic acid molecule is double stranded - the strands may be complementary or partially complementary. Moreover, steps (3)-(4) may be carried out subsequently or contemporaneously. The following figures further explain the embodiments of the invention. The figures are, however, not to be construed to limit the invention.

### Short Description of the Figures

- Figure 1:: is a schematic illustration of the method of the invention.
- Figure 2:: illustrates the principle of the method using dUMP as the marker nucleotide and employing UDG, a class II AP endonuclease and a dRPase for the introduction of single-stranded breaks at the marker nucleotides.
- Figure 3:: illustrates the principle of the method using dUMP as the marker nucleotide and employing UDG, a class I AP endonuclease and a class II AP endonuclease for the introduction of single-stranded breaks at the marker nucleotides.
- Figure 4:: illustrates the principle of the method using dUMP as the marker nucleotide and employing UDG, Endo VIII or Fpg, and a class II AP endonuclease or a T4 polynucleotide kinase for the introduction of single-stranded breaks at the marker nucleotides.
- Figure 5:: illustrates the principle of the method using rNMP as the marker nucleotide and employing RNase H for the introduction of single-stranded breaks at the marker nucleotides.

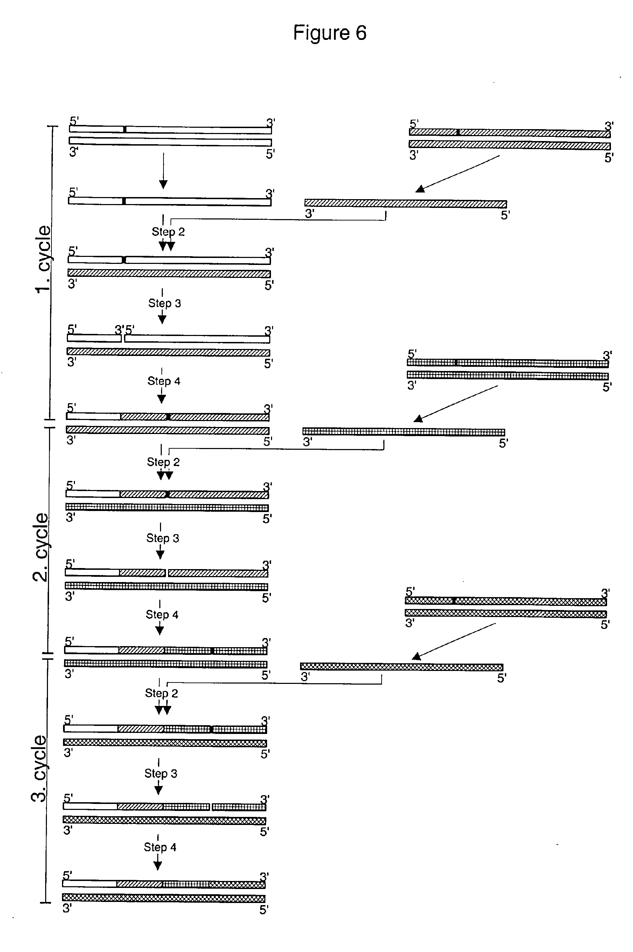

- Figure 6:: is a schematic illustration of the method of the invention employing three cycles.

### Detailed Description of the Invention

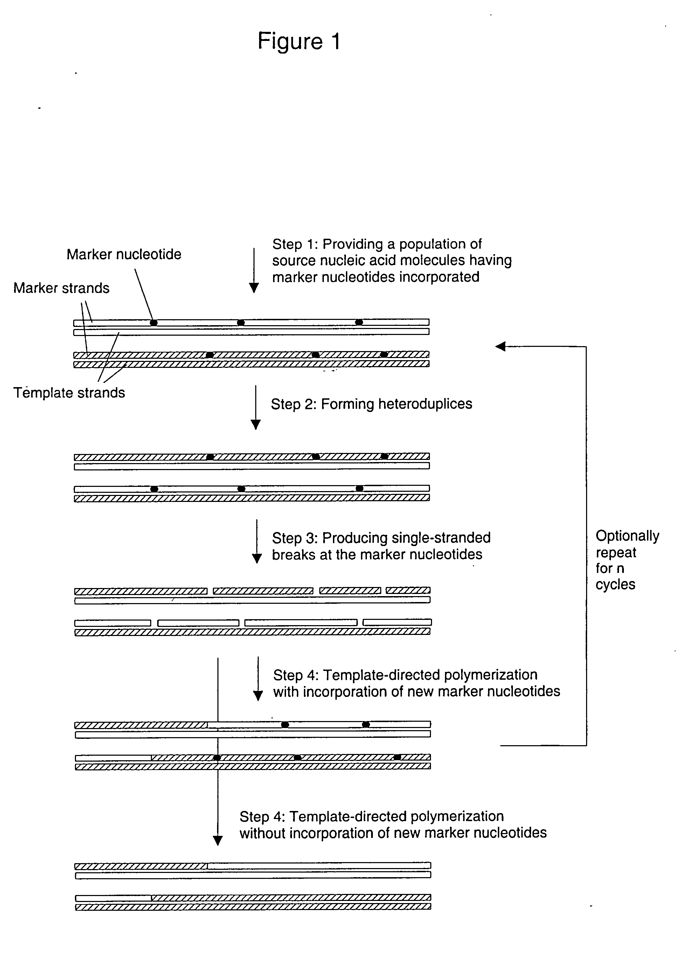

As set forth above, embodiment (A) of the invention relates to a method for the production of nucleic acids with modified properties, i.e., polynucleotides consisting of stochastically combined parts of the source nucleic acids. Said embodiment will be described in more detail with reference to figure 1, which schematically shows a possible variant of the method of the invention.

Depending on the requirements, the method of the invention permits both an incidental and a controlled new combination of heterologous sequence segments. By adjusting the probability of incorporation of marker nucleotides the average distance between recombination sites can be controlled. Distances down to one nucleotide are possible using appropriate ratios of nucleotides and marker nucleotides. This is hardly achieved with any of the before mentioned methods. In addition, the frequency of recombination can be controlled in a wide range by adjusting the number of cycles and the average recombination distance per cycle. Such a control of the recombination frequency may also be achieved by means of the method described in WO 01/34835 and the method described in WO 98/42728 that relys on a PCR with strand exchange. It is at least in part achieved by means of the methods described in WO 95/22625 and WO 01/29211. The method described in WO 98/42728 that relies on random priming provides no means to control the recombination frequency.

Another aspect of methods for homologous recombination is their requirement for a certain degree of homology between the source nucleic acids to be recombined. The methods described in WO 95/22625, WO 98/42728 and WO 01/29211 all rely on the annealing of short sequence segments between the sites of recombination. In case that recombination events shall be distributed evenly over the entire nucleic acid sequence, this means that the entire source nucleic acid sequences have to provide sufficient homology to enable annealing of short nucleic acid segments. Regions within the nucleic acid sequences with lower homology permit said annealing and accordingly interrupt the recombination reaction. In contrast, the method described in WO 01/34835 as well as the method of the invention employ annealing of full length nucleic acid sequences to produce heteroduplices which are subjected to the recombination process. Thereby, also regions with rather low homology within the nucleic acid sequence do not interrupt the recombination and an overall lower homology is tolerated when compared to the above mentioned methods.

Hence, the method of the invention is characterized by a combination of advantages which could not be achieved with any of the methods disclosed so far.

Products resulting from each individual cycle according to the method of the invention are semi-conservative, single-stranded nucleic acid molecules, since - depending on the embodiment - a longer or shorter sequence segment was maintained at one side of the marker nucleotide incorporation site while the sequence segment on the other side of the marker nucleotide incorporation site was synthesized newly with the information of the template strand.

The term "marker nucleotides" in accordance with the present invention means any nucleic acid monomer that is suited to be incorporated into a polynucleotide and that can be used as a marker to introduce single-stranded breaks at the corresponding position in order to provide intramolecular starting points for a template-directed polymerization reaction. Preferably, marker nucleotides are analogous of standard nucleotides that can be recognized specifically by a chemical reaction or by enzymatic treatment.

In a preferred embodiment, more than one cycle comprising the aforementioned steps (1) to (4) is completed, i.e. at least two, preferably at least ten, more preferably at least twenty and most preferably at least fifty cycles. In this embodiment, the template directed single-strand synthesis in step (4) is done with incorporation of marker nucleotides, thereby introducing in each cycle the marker nucleotides for the next cycle. Then, preferably, the last cycle is done without incorporation of marker nucleotides in order to produce double-strands free from marker nucleotides that can be processed further.

The cyclic application of the method of the invention makes it possible to produce nucleic acid molecules comprising multiple recombined sequence segments from different source nucleic acids. In particular, the cyclic application makes it possible to combine several heterologous sequence segments which each other. Moreover, it is possible to control the recombination frequency for each polynucleotide strand by the number of cycles. With cyclic application, the average distance between the new combinations can be controlled by the probability of incorporating marker nucleotides in each cycle.

In particular, the average distance between the starting points of the template-directed synthesis according to step (4) in each of two consecutive cycles is controlled by adjusting the probability of incorporating marker nucleotides in step (4) of the first of the two consecutive cycles. The probability of incorporating marker nucleotides can be controlled by adjusting the ratio of concentrations of marker nucleotides to standard nucleotides. Preferably, the probability of incorporating marker nucleotides is chosen to be lower than one and higher than the reciprocal of the length of the source nucleic acids in base pairs. It is noteworthy that, whenever more than one marker nucleotide is incorporated per polynucleotide strand, only the marker nucleotide incorporated next to the starting point of the template-directed polymerization determines the distance between recombination sites. All other marker nucleotides are removed without consequences (cf. Fig. 1).

In a preferred embodiment, the nucleic acid molecules in the population of source nucleic acid molecules provided according to step (1) are double strands and the marker nucleotides are incorporated within the nucleic acid sequence in both strands. Here, both strands are accessible for the production of single-stranded breaks according to step (3) and, therefore, both strands are subjected to recombination and, at the same time, can serve as template strands.

In another preferred embodiment, the nucleic acid molecules in the population of source nucleic acid molecules provided according to step (1) are double strands and the marker nucleotides are incorporated within the nucleic acid sequence in only one of both strands (marker strand or sense strand). Accordingly, only one of both strands is accessible for the production of single-stranded breaks according to step (3) and, therefore, only one of both strands is subject for recombination. The other strand serves only as a template during the whole process (template strand or antisense strand).

In a particularly preferred embodiment, said double strands consisting of a marker strand and a template strand are produced by PCR using one primer having at least one marker nucleotide incorporated and a second primer without having any marker nucleotides incorporated.

In another particularly preferred embodiment, said double strands consisting of a marker strand and a template strand are produced by annealing two single strands, each of which is produced by asymmetric PCR using only one primer, the marker strand being produced with incorporation of marker nucleotides during the polymerization step, while the template strand is produced without incorporation of marker nucleotides during the polymerization step.

In another preferred embodiment, the marker nucleotides incorporated in the population of source nucleic acid molecules provided according to step (1) are incorporated next to the 5'-end and the recombination site defined by the incorporation site of marker nucleotides and the corresponding single-stranded break according to step (3) gets closer to the 3'-end of the polynucleotide molecules with increasing cycle number.

In another preferred embodiment, when more than one cycle is completed, the probability of incorporating marker nucleotides is altered from cycle to cycle. For example, this can be done by altering the ratio of concentrations of marker nucleotides and corresponding standard nucleotides. In this way, the distance between recombination sites can be controlled regioselectively.

The population of source nucleic acid molecules provided according to step (1) of the method of the invention can be any population of nucleic acid molecules comprising at least two kinds of polynucleotides, consisting of homologous and heterologous segments. Preferably, two of these polynucleotides each have at least one homologous and two heterologous sequence segments when compared with each other. The term "population of nucleic acid molecules" refers to any kind of nucleic acid, e.g. single-stranded DNA, double-stranded DNA, single-stranded RNA, double-stranded RNA, double-stranded hybrids of DNA and RNA, or mixtures of any of these. In principle, the method may also be used for similarly constructed, artificial polymers. The term "homologous segments" denotes segments which are identical or complementary on two or more nucleic acid molecules, i.e. which have the same information at corresponding positions. The term "heterologous segments" means segments which are not identical or complementary on two or more nucleic acid molecules, i.e. which have different information at corresponding positions. The term "information" or "genotype" of a nucleic acid molecule is the sequential order of various monomers in a nucleic acid molecule. A heterologous sequence segment has preferably a length of at least one nucleotide, but may also be much longer. For example, a heterologous sequence segment may have a length of two nucleotides or three nucleotides, e.g. a codon. In principle, there is no upper limit as regards the length of the heterologous segment. Nevertheless, the length of a heterologous segment should not exceed 1,000 nucleotides, preferably it should not be longer than 500 nucleotides, more preferably not longer than 200 nucleotides and most preferably not longer than 100 nucleotides. Such longer sequence segments may, for example, be the hypervariable regions of a sequence encoding an antibody, domains of a protein, genes in a gene cluster, regions of a genome, etc. Preferably, the heterologous segments are sequence segments in which the nucleic acid molecules differ in single bases. Heterologous segments, however, may also be based on the fact that a deletion, duplication, insertion, inversion, addition or similar is present or has occurred in a nucleic acid molecule.

According to the invention, the nucleic acid molecules provided according to step (1) of embodiment (A) have preferably at least one homologous and at least two heterologous sequence segments. More preferably, however, they have a plurality of homologous and heterologous segments. In principle, there is no upper limit to the number of homologous and heterologous segments. A population of source nucleic acid molecules according to the invention may consist of (i) gene variants each carrying one or more point mutations at various positions, or (ii) of gene homologous obtained from different species providing sufficient homology to produce - at least partially - heteroduplices, or (iii) of gene variants each carrying one or more randomized cassettes such as antibody gene libraries. This enumeration is, however, not to be construed to limit the invention.

The heterologous segments in the population of nucleic acid molecules provided according to step (1) of the method are each interrupted by homologous segments. The homologous segments preferably have a length of at least 5, more preferably of at least 10 and most preferably of at least 20 nucleotides. Like the heterologous segments, the homologous segments, too, may be much longer and, in principle, there is no upper limit to their length. Preferably, their length should not exceed 5,000 nucleotides, more preferably not longer than 2,000 nucleotides and most preferably not longer than 1,000 nucleotides.

In a particularly preferred embodiment of the method of the invention related nucleic acid sequences are used for providing a population of source nucleic acid molecules according to step (1). In this context, the term "related" means polynucleotides which have both homologous and heterologous segments among each other. Related nucleic acid molecules may originate from a procedure to introduce random point mutations into a source nucleic acid sequence. This introduction of point mutations can be achieved by the inherent erroneous copying process alone, but also by the purposeful increase of the inaccuracy of the polymerase used (e.g. by defined non-balanced addition of the monomers, by addition of base analogues, by error-prone PCR, by polymerases with very high error rate), by chemical modification of polynucleotides after synthesis, by the complete synthesis of polynucleotides under at least partial application of monomer mixtures and/or of nucleotide analogues, by errornous replication in vivo (e.g. by viruses having high error rates, by bacterial mutator strains, by bacteria under UV irradiation, etc.), as well as by a combination of two or more of these methods. Related nucleic acid molecules may also be nucleic acid molecules, that have been subjected to an alternative nucleic acid variation method, such as the random truncation, insertion, deletion or inversion of sequence segments or the introduction of randomized sequence segments. Related nucleic acid molecules may also be nucleic acid sequences of the distribution of mutants of a quasi-species. A "quasi-species" is a dynamic population of related molecule variants (mutants) which is formed by faulty replication and subsequent selection (WO 92/18645). Alternatively, related nucleic acid molecules may be nucleic acid sequences isolated from natural sources that have a sufficient degree of homology to form heteroduplices according to step (2) of the method. For example, analogous genes or gene fragments isolated from genomes of evolutionary related species can be employed. Any of these related nucleic acid molecules may be used directly or be subjected to a screening and/or selection procedure before application of the recombination procedure, the selection and/or screening procedure selecting those nucleic acid molecules that have a certain phenotype. The term "phenotype of a nucleic acid molecule" denotes the sum of functions and properties of the nucleic acid molecule and of the transcription or translation products encoded by the nucleic acid molecule.

The incorporation of marker nucleotides according to step (1) and, where applicable, according to step (4) is achieved by using a template directed polymerase reaction or by chemical synthesis of oligonucleotides. Preferably, the incorporation of marker nucleotides according to step (4) is done by using a template-directed polymerase reaction.

For said template-directed polymerase reaction according to step (4) of the method any enzyme with template directed polynucleotide-polymerization activity can be used which is able to polymerize polynucleotide strands starting from the 3'-end. A vast number of polymerases from the most varied organisms and with different functions have already been isolated and described. With regard to the kind of the template and the synthesized polynucleotide, a differentiation is made between DNA-dependent DNA polymerases, RNA-dependent DNA polymerases (reverse transcriptases), DNA-dependent RNA polymerases and RNA-dependent RNA polymerases (replicases). With regard to temperature stability, a differentiation is made between non-thermostable (37°C) and thermostable polymerases (75-95°C). In addition, polymerases differ with regard to the presence of 5'-3'- and 3'-5'-exonucleolytic activity.

When both, the template strand and the marker strand consist of DNA, DNA-dependent DNA polymerases are preferably used. In particular, DNA polymerases with a temperature optimum of exactly or around 37°C are used. These include, for instance, DNA polymerase I from E. coli, T7 DNA polymerase from the bacteriophage T7 and T4 DNA polymerase from the bacteriophage T4 which are each traded by a large number of manufacturers. The DNA polymerase I from E. coli (holoenzyme) has a 5'-3' polymerase activity, a 3'-5' proofreading exonuclease activity and a 5'-3' exonuclease activity. The enzyme is used for in vitro labelling of DNA by means of the nick-translation method (J. Mol. Biol. 113 (1977), 237-251). In contrast to the holoenzyme, the Klenow fragment of DNA polymerase I from E. coli does not have a 5'-exonuclease activity, just like the T7 DNA polymerase and the T4 DNA polymerase. Therefore, these enzymes are used for so-called filling-in reactions or for the synthesis of long strands (Biochemistry 31 (1992), 8675-8690, Methods Enzymol. 29 (1974), 46-53). The 3'-exo(-) variant of the Klenow fragment of DNA polymerase I from E. coli does not have the 3'-exonuclease activity. This enzyme is often used for DNA sequencing according to Sanger (Proc. Natl. Acad. Sci. USA 74 (1977), 5463-5467). Apart from these enzymes, there is a plurality of other 37°C DNA polymerases with different properties which can be employed in the method of the invention.

Moreover, thermostable DNA polymerases can be used for the method of the invention. Preferably, the most widespread thermostable DNA polymerase that has a temperature optimum of 75°C and is still sufficiently stable at 95°C, the Taq DNA polymerase from Thermus aquaticus, can be used. Taq DNA polymerase is commercially available from various manufacturers. Taq DNA polymerase is a highly-processive DNA polymerase without 3'-exonuclease activity. It is often used for standard PCRs, for sequencing reactions and for mutagenic PCRs (PCR Methods Appl. 3 (1994), 136-140, Methods Mol. Biol. 23 (1993), 109-114). However, several other thermostable DNA polymerases can be employed. The Tth DNA polymerase from Thermus thermophilus HB8 and the Tfl DNA polymerase from Thermus flavus have similar properties. The Tth DNA polymerase additionally has an intrinsic reverse transcriptase (RT) activity in the presence of manganese ions (Biotechniques 17 (1994), 1034-1036). Among the thermostable DNA polymerases without 5'- but with 3'-exonuclease activity, numerous of them are commerically available: Pwo DNA polymerase from Pyrococcus woesei, Tli, Vent or DeepVent DNA polymerase from Thermococcus litoralis, Pfx or Pfu DNA polymerase from Pyrococcus furiosus, Tub DNA polymerase from Thermus ubiquitous, Tma or UITma DNA polymerase from Thermotoga maritima. Polymerases without 3'-proofreading exonuclease activity are used for amplifying PCR products that are as free from defects as possible. With the Stoffel fragment of Taq DNA polymerase, with Vent-(exo-) DNA polymerase and Tsp DNA polymerase thermostable DNA polymerases without 5'- and without 3'-exonucleolytic activity are available.

When RNA is used as the template strand nucleic acid and DNA as the marker strand nucleic acid, RNA-dependent DNA polymerases (reverse transcriptases) can be employed. Among the reverse transcriptases, preferably, the AMV reverse transcriptase from the avian myeloblastosis virus, the M-MuLV reverse transcriptase from the Moloney murine leukemia virus or the HIV reverse transcriptase from the human immunodeficiency virus is used. All three enzymes are traded by various manufacturers. Like the HIV reverse transcriptase, the AMV reverse transcriptase has an associated RNase-H activity. This activity is significantly reduced in M-MuLV reverse transcriptase. Both the M-MuLV and the AMV reverse transcriptase do not have a 3'-exonuclease activity. Furthermore, a thermostable reverse transcriptase can be used. Then the Tth-DNA polymerase from Thermus thermophilus with intrinsic reverse transcriptase activity is particularly preferred.

When DNA is used as the template strand nucleic acid and RNA as the marker strand nucleic acid, DNA-dependent RNA polymerases may be employed. Preferably, the RNA polymerase from E. coli, the SP6-RNA polymerase from Salmonella typhimurium LT2 infected with the bacteriophage SP6, the T3-RNA polymerase from the bacteriophage T3 or the T7-RNA polymerase T7 from the bacteriophage T7 is used.

In a preferred embodiment of the method, DNA is used as nucleic acid and deoxyuridine triphosphate (dUTP) is used as the marker nucleotide. Here, the incorporation of marker nucleotides according to step (1) and, where applicable, according to step (4) is achieved by using dUTP in combination with the four standard deoxynucleoside triphosphates (dNTPs; deoxyadenosine triphosphate, dATP; deoxyguanosine triphosphate, dGTP; deoxythymidine triphosphate, dTTP; deoxycytidine triphosphate, dCTP) in the template-directed polymerase reaction. The ratio of the dUTP to the dTTP concentration in this reaction can be chosen in a wide range in order to control marker nucleotide incorporation probability and, thereby, control the recombination distances. The exact ratio has to be adapted to the discrimination rate between dTTP and dUTP of the polymerase used in the template-directed polymerase reaction as well as to the desired average distance between recombination sites. The discrimination rates between dTTP and dUTP for a few of the aforementioned polymerases are: Taq DNA polymerase (Vₘₐₓ/Kₘ for the incorporation of dTTP) / (Vₘₐₓ/Kₘ for the incorporation of dUTP) = 1.2; Klenow DNA polymerase = 1.6; Vent DNA polymerase = 1.4; MMLV reverse transcriptase = 6.3 (J. Biol. Chem. 275 (2000) 40266). As an example, when Taq DNA polymerase from Thermus aquaticus is used and average distances in the range of 20 to 60 nucleobases are desired, the concentration ratio of dTTP to dUTP should, preferably, be lower than 100,000 and be higher than 0.001. More preferably the ratio should be lower than 1,000 and be higher than 0.1. Most preferably, the concentration ratio of dTTP to dUTP should be in the range of 10.

In another preferred embodiment the nucleic acids used are DNA and 8-oxo-de-oxyguanosine triphosphate (8-oxo-dGTP) is used as the marker nucleotide in combination with the four standard dNTPs in the template directed polymerase reaction. The marker incorporation probability and, thereby, the distance between recombination sites can be controlled by chosing an appropriate concentration ratio between 8-oxo-dGTP and dGTP. As an example, when Taq DNA polymerase from Thermus aquaticus is used and average distances in the range of 20 to 60 nucleobases are desired, the concentration ratio of 8-oxo-dGTP to dGTP in this reaction should preferably be chosen between 100,000 and 10. More preferably, the concentration ratio should be chosen between 10,000 and 100. Most preferably, the concentration ratio should be in the range of 1,000.

In another preferred embodiment marker nucleotides with one of the following modified bases are used in combination with the four standard dNTPs in the template directed polymerase reaction: 3-methyladenine, 7-methyladenine, 3-methylguanine, 7-methylguanine, 7-hydroxyethylguanine, 7-chloroethylguanine, O2-alkylthymine, O2-alkylcytosine, 5-fluorouracil, 2,5-amino-5-formamidopyrimidine, 4,6-diamino-5-formamidopyrimidine, 2,6-diamino-4-hydroxy-5-formamidopyrimidine, 5-hydroxycytosine, 5,6-dihydrothymine, 5-hydroxy-5,6-dihydrothymine, thymine glycol, uracil glycol, isodialuric acid, alloxan, 5,6-dihydrouracil, 5-hydroxy-5,6-dihydrouracil, 5-hydroxyuracil, 5-formyluracil, 5-hydroxymethyluracil, hypoxanthine, 1,N6-ethenoadenine, or 3,N4-ethenocytosine. For the polymerase reaction any enzyme with template directed polynucleotide-polymerization activity can be used which is able to incorporate these marker nucleotides.

In another preferred embodiment the marker strand nucleic acid is DNA, and one, two, three or all four ribonucleoside triphosphates (rNTPs) are used in combination with the four standard dNTPs in the template directed polymerase reaction. The concentration ratio of the rNTP to the corresponding dNTP in this reaction can be used to control the marker incorporation probability and, thereby, the distance between recombination sites. Discrimination ratios (Vₘₐₓ/Kₘ for the incorporation of dNTP) / (Vₘₐₓ/Kₘ for the incorporation of rNTP) for Taq DNA polymerase are: dUTP/rUTP = 1,500,000, dCTP/rCTP = 24,000; for Klenow DNA polymerase: dUTP/rUTP = 130,000, dCTP/rCTP = 3,100; for Vent DNA polymerase: dUTP/rUTP = 10,000, dCTP/rCTP = 2,000; and for MMLV reverse transcriptase: dUTP/rUTP = 21,000, dCTP/rCTP = 1,100 (J. Biol. Chem. 275, (2000) 40266). As an example, when Vent DNA polymerase is used in combination with rCTP as the marker nucleotide, and average distances in the range of 20 to 60 nucleobases are desired, the concentration ratio of rCTP to dCTP should preferably be lower than 10,000 and higher than 1. More preferably, the ratio should be lower than 1,000 and higher than 10. Most preferably the ratio should be in the range of 100.

The formation of double-stranded heteroduplices according to step (2) of the method of the invention is preferably achieved by hybridization of the homologous segments of the source nucleic acid molecules. The term "heteroduplices" means double strands with at least one homologous and at least two heterologous segments. By using a population of nucleic acid sequences with heterologous segments, heteroduplices are formed with a statistical probability which corresponds to the relative frequency of sequence variants in the population. Starting out, for example, from an equimolar mixture of two variants having two heterologous segments, a heteroduplex statistically occurs with every second double-stranded nucleic acid. If the number of variants is markedly higher than the relative frequency of individual variants, heteroduplices are formed almost exclusively.

Hybridization of homologous segments of the source nucleic acids to form heteroduplices is carried out according to methods known to the person skilled in the art. In a preferred embodiment the source nucleic acid molecules are single-stranded and the hybridization is achieved by combining said single strands and adjusting reaction conditions which promote the annealing of homologous nucleic acids, e.g. by lowering of the temperature or adjusting the salt concentration. In another preferred embodiment, the source nucleic acid molecules are double-stranded and the hybridization is achieved by melting the double strands under appropriate conditions, e.g. at temperatures higher than the melting temperature of the double strand, and allow the strands to re-anneal, e.g. by lowering the temperature below the melting temperature of the double strand.

The production of single-stranded breaks at the positions of incorporated marker nucleotides according to step (3) of the invention is preferably achieved by chemical or enzymatic reactions. The term "breaks" means nicks or gaps in a nucleic acid strand that can serve as starting points for a template-directed polymerase reaction.

In a preferred embodiment, the single-stranded break is achieved by removing the marker nucleotide by the action of one or more enzymes leading to a single nucleotide gap and a free 3'-OH residue on the 5' side of said gap, the free 3'-OH being extendable by a polymerase according to step (4) of the method.

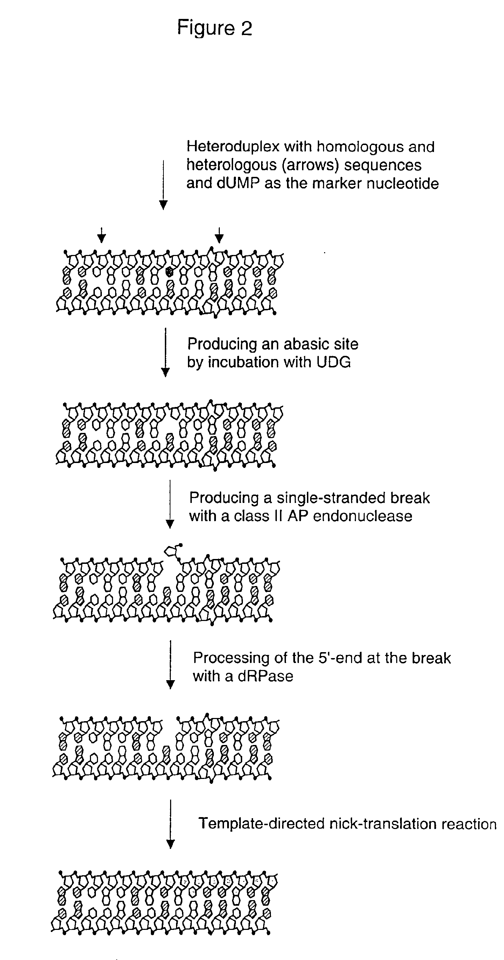

In a particularly preferred embodiment, when DNA is the nucleic acid and dUTP is used as marker nucleotide, the uracil base of the incorporated marker uridine residues is separated from the ribose by action of an uracil-DNA glycosylase (UDG, Figures 2 - 4). A large number of different UDGs isolated from various species has been described (Rev. Biochem. Tox. 9 (1988) 69; Mutat. Res. 460 (2000) 165). UDGs are involved in a base-excision pathway initiated by deamination of the DNA base cytosine leading to uracil or by misincorporation of uridine during DNA replication. The use of UDGs in PCR-carry-over-prevention has been described (Gene 93 (1990) 125). UDG from E. coli is commercially available in the engineered and in the non-engineered form by various manufacturers. E. coli UDG efficiently hydrolyzes uracil from single-stranded or double-stranded DNA, but not from dUTP. The minimal substrate for UDG was found to be pd(UN)p (Biochemistry 30 (1991) 4055). The reaction can be started e.g. by changing the buffer conditions or the temperature or by adding the UDG, and can be stopped, for instance, by changing the buffer conditions or the temperature or by adding an UDG inhibitor. The separation of the uracil bases from DNA containing uridine residues results in apyrimidinic sites (AP sites).

In another particularly preferred embodiment, when DNA is the nucleic acid and 8-oxo-dGTP is used as marker nucleotide, the 8-oxo-guanine base is separated from the ribose using formamidopyrimidine-DNA glycosylases (Fpg) (EMBO J. 6 (1987) 3177). The reaction can be started e.g. by changing the buffer conditions or the temperature or by adding the enzyme and can be stopped, for instance, by changing the buffer conditions or temperature or by adding an inhibitor. In addition to its formamidopyrimidine-glycosylase activity, this protein also has a nicking activity that cleaves via a α,β-elimination both the 5'- and 3'-phosphodiester bonds at an AP site (Biochem. J. 262 (1989) 581). Thus the treatment of a polynucleotide molecule containing the 8-oxo-GMP residues leads to gaps of a single nucleotide with a phosphate group both at the 5'- and 3'-end.

In another particularly preferred embodiment any other DNA N-glycosylase which detects one of the aforementioned modified bases is employed. E. coli alkylbase-DNA glycosylase (alkA gene product, Mol. Gen. Genet. 197 (1984) 368), for example, separates the bases from 3-methyladenosine, 7-methyladenosine, 3-methylguanosine, 7-methylguanosine, 7-hydroxyethylguanosine, 7-chloroethylguanosine, O2-alkylthymidine, O2-alkylcytidine, hypoxanthosine, 1,N6-ethenoadenosine or 3,N4-ethenocytidine. E. coli endonuclease III (Biochem. J. 242 (1987) 565), as an alternative, separates the bases from 5-hydroxycytidine, 5,6-dihydrothymidine, 5-hydroxy-5,6-dihydrothymidine, thymidine glycol, uridineglycol, alloxan, 5,6-dihydrouridine, 5-hydroxy-5,6-dihydrouridine or 5-hydroxyuridine. Endonuclease III has in addition to its DNA N-glycosylase activity an AP lyase activity which cleaves at the 3'-end bond of an AP site via β-elimination (Nucl. Acid. Res. 16 (1988) 1135). Thus the treatment of a nucleic acid molecule containing the aforementioned substrate residues for endonuclease III leads to nicks with a α,β-unsaturated aldehyde (*trans*-4-hydroxy-2-pentenal-5-phosphate) at the 3'-end and a phosphate group at the 5'-end.

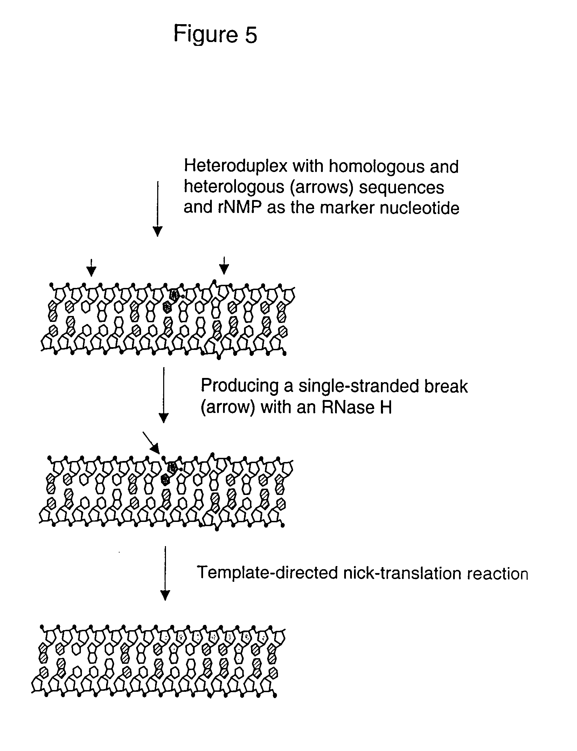

In another particularly preferred embodiment, when DNA is the nucleic acid and one or more rNTPs are used as marker nucleotides, the rNMP residues incorporated in a DNA double strand can be recognized by a ribonuclease H (RNase H, Figure 5). Preferably, RNase H1 from K562 human erythroleukemia cells is used, that cleaves at the 5'-site of an RNA segment in the DNA strand consisting of one or more ribonucleotide residues (J. Biol. Chem. 266 (1991) 6472). This reaction leads to a nick with a 5'-p-rNMP residue at one side and a free 3'-OH group at the other side. Alternatively, other RNases H can be employed, e.g. E. coli RNase H or the RNase H activity of reverse transcriptases. The reaction can be started e.g. by changing the buffer conditions or the temperature or by adding the enzyme and can be stopped, for instance, by changing the buffer conditions or temperature or by adding an inhibitor.

In a preferred embodiment the AP site resulting from the action of a DNA N-glycosylase is cleaved by a class II AP endonuclease (Figure 2). In particular, Endonuclease IV (J. Biol. Chem. 252 (1977) 2808) or Exonuclease III (J. Biol. Chem., 239 (1964) 242) can be used for this reaction. The incubation of a polynucleotide molecule containing AP sites with these enzymes leads via hydrolysis to a nick with a 5'-deoxyribosephosphate (dRp) group at one side and a free 3'-OH group at the other side (Nucl. Acid. Res. 18 (1990) 5069).

In a particularly preferred embodiment the 5'-dRp group resulting from the action of a class II AP endonuclease is cleaved by an enzyme showing deoxyribose-phosphatase activity (dRpasen). For this reaction, a multitude of enzymes can be employed. For example: E. coli exonuclease I (Nucl. Acid. Res. 20 (1992) 4699), E. coli RecJ protein (Nucl. Acid. Res. 22, 1994, 993); E. coli endonuclease III (Nucl. Acid. Res. 17, 1989, 6269); E. coli formamidopyrimidine-DNA glycosylase (Fpg, J. Biol. Chem. 267, 1992, 14429); E. coli endonuclease VIII (J. Biol. Chem. 272, 1997, 32230); T4 endonuclease V (Biochemistry 32, 1993, 8284); T4 DNA ligase (J. Biol. Chem. 273, 1998, 7888); T7 DNA ligase (J. Biol. Chem. 273, 1998, 7888) or DNA polymerase I, T7 DNA polymerase and MMLV reverse transcriptase (J. Biol. Chem. 275, 2000, 12509).

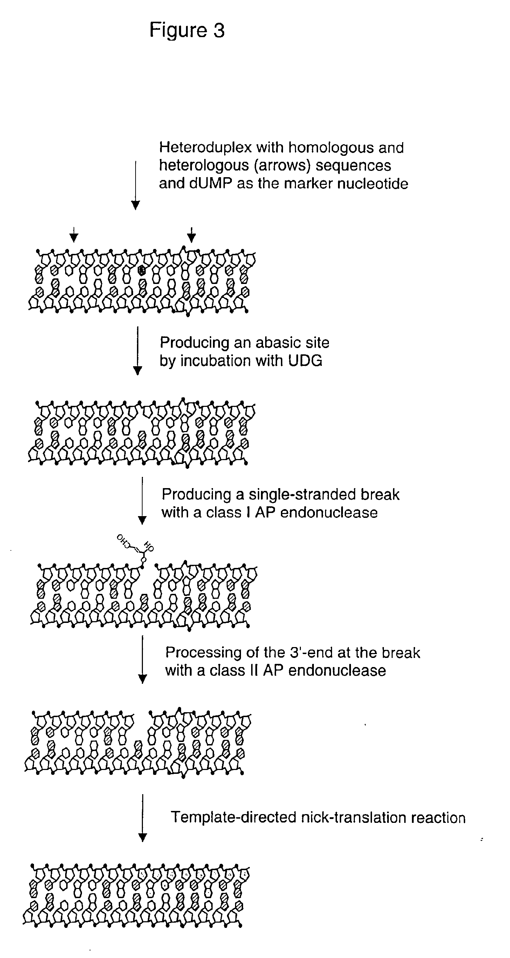

In another preferred embodiment the AP site resulting from the action of a DNA N-glycosylase is cleaved by a class I AP endonuclease (Figure 3). For this reaction, E. coli endonuclease III (Biochem. J. 242, 1987, 565-573) or T4 endonuclease V (Mutat. Res. 459, 2000, 43-53) can be employed. The incubation of a polynucleotide molecule containing AP sites with these enzymes leads via β-elimination to a nick with a α,β-unsaturated aldehyde (*trans*-4-hydroxy-2-pentenal-5-phosphate) at the 3'-end and a phosphate group at the 5'-end (FEBS Lett. 178, 1984, 223; Nucl. Acid. Res. 16, 1988, 1135). The 3'-aldehyde has to be removed by a class II AP endonuclease such as exonuclease III or endonuclease IV (Biochem. J. 242, 1987, 565) resulting in a free 3'-OH group.

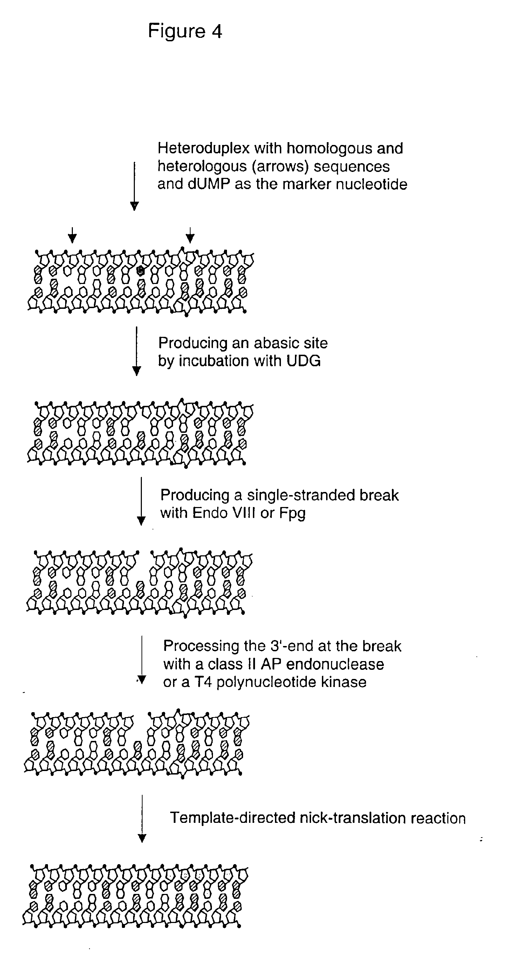

In a preferred embodiment the AP site resulting from the action of a DNA N-glycosylase is cleaved by an AP lyase which cleaves at the AP site via a α,β-elimination (Figure 4). E. coli endonuclease VIII (J. Biol. Chem. 272, 1997, 32230) and E. coli formamidopyrimidine-DNA glycosylase (Fpg, J. Biol. Chem. 267, 1992, 14429) can be employed for this purpose. The incubation of a DNA double strand containing AP sites with these enzymes leads to gap of one nucleotide with a 3'-phosphate residue at one side of the gap and a 5'-phosphate residue at the other side of the gap. Afterwards, the 3'-phosphate group is removed by a class II AP endonuclease, as for example Exonuclease III or Endonuclease IV (J. Biol. Chem. 258, 1983, 15198) or by T4 polynucleotide kinase (Biochemistry 16, 1977, 5120) resulting in a free 3'-OH group.

In another preferred embodiment the marker strand consisting of DNA and containing rNMP residues is cleaved by alkaline hydrolysis. This reactions leads to a nick with a 2'- or 3'-rNMP at the 3'-end and an OH group at the 5'-end. The reaction can be started and stopped by changing the pH.

In another preferred embodiment the 2'- or 3'-rNMP at the 3'-end of a nick resulting from the alkaline hydrolysis of a DNA polynucleotide containing rNMPs is removed by a class II AP endonuclease. Preferably, Exonuclease III or Endonuclease IV are used, resulting in a free 3'-OH group.

According to step (4), the free 3'-OH group at a nick or gap resulting from one or more of the aforementioned reactions is extended with a template directed polymerase reaction with or without the incorporation of additional marker nucleotides.

In a preferred embodiment the remaining part of the marker strand 3' of the single strand break, in particular strands containing a 5'-dRp group resulting from the action of a class II AP endonuclease, are bound with a surplus of the corresponding complementary strands and are thereby removed from the template strand. Then, any kind of polymerase can be employed to extend the 3'-OH group by template-directed polymerization.

In another preferred embodiment the remaining part of the marker strand 3' of the single strand break is removed from the template strand by employing a polymerase showing strong strand displacement properties. Preferably, Vent DNA polymerase or Klenow DNA polymerase are employed for this purpose.

In another preferred embodiment the remaining part of the marker strand 3' of the single strand break is removed from the template strand by a 5'-exonuclease activity. For this purpose, any polymerase showing a 5'-exonuclease activity can be employed. Preferably, Taq DNA polymerase or Tth DNA polymerase are used. Alternatively, 5'-3' exonucleases can be used in combination with any polymerase. Then, preferably, Lambda Exonuclease (Gene Amplification and Analysis 2,1981, 135) or T7 Exonuclease (Nucl. Acid. Res. 5, 1978, 4245) are used.

Embodiment (B) of the invention relates to a kit containing instructions for carrying out the method embodiment (A) of the invention. Preferably, said kit contains at least one, preferably at least two, and most preferably all of the following components:
(i) buffer for producing double-stranded polynucleotides;
(ii) marker nucleotides for incorporation in the polynucleotide molecules;
(iii) agents permitting the single-stranded breaks at the incorporated marker nucleotides;
(iv) buffers for carrying out the incorporation of the marker nucleotides and producing the single-stranded breaks;
(v) agents permitting the template-directed polymerization of a polynucleotide strand starting form the single-stranded break; and
(vi) buffer for carrying out the polymerization reaction.

The invention is further explained by the following experimental protocols, which are, however, not to be construed to limit the invention.

### Experimental Protocols

Protocol A: Generating single recombination events per gene that are randomly distributed
1. Provide partially homologous and heterologous genes to be recombined. Amplify the genes by PCR introducing an *Eco* RI restriction site at the one end and a *Hind* III restriction site at the other end.
2. Incubate 1 µg of each PCR product and 1 µg of pUC18 vector with 1 U *Eco* RI (e.g. NEB) and 1 U *Hind* III (e.g. NEB) in *Eco* RI reaction buffer (100 mM Tris-HCl, pH 7.5; 50 mM NaCl; 10 mM MgCl₂; 0.025 % (v/v) Triton® X-100) for 2 h at 37 °C. Heat inactivate the enzymes for 20 min at 65 °C. Purify the cleavage products e.g. with QiaQuick (Qiagen).
3. Ligate the PCR products into the pUC18 vector using 200 fmol vector, 600 fmol insert, 1 µl of 10X Ligation Buffer (500 mM Tris-HCI, pH 7.5; 100 mM MgCl₂; 100 mM DTT; 10 mM ATP, 250 µg/ml BSA), 5 Weiss Unit of T4 DNA ligase (e.g. NEB) ad 10 µl aqua dest. Incubate 1 h at room temperature and heat inactivate the enzyme for 10 min at 65 °C. Transform *E*. *coli* XL1-Blue with the ligated vector, e.g. by electroporation. Make plasmid preparations from positive clones using e.g. Qiagen Mini Plasmid Prep Kits.
4. Amplify the inserted genes with a PCR using the primers:
   pUC-left: 5'-CCAGTCACGACGTTGTAAAACG-3' SEQ ID NO:1
   pUC-right: 5'-TAACAATTTCACACAGGAAACAGC-3' (SEQ ID NO:2 by mixing 10 µl 10X PCR buffer (200 mM Tris-HCI, pH 8.75; 100 mM KCI; 100 mM (NH4)₂ SO₄; 20 mM MgCl₂; 1 % (v/v) Triton® X-100; 1 mg/ml BSA), 10 fmol template vector, 100 pmol pUC-left, 100 pmol pUC-right, 200 µM dNTPs, 2 U Pfu DNA polymerase (e.g. Stratagene) ad 100 µl aqua dest. and using the following cycler protocol: 1' 94 °C; 30 cycles consisting of 1' 94 °C, 1' 50 °C, 1.5' 72 °C; 2' 72 °C. Purify the PCR products, e.g. with QiaQuick®.
5. Make a set of asymmetric PCRs with the mixed PCR-products as templates varying the added dUTP concentration (e.g. 0.2 µM; 1 µM; 5 µM; 25 µM; 100 µM dUTP) by mixing 10 µl 10X PCR buffer (100 mM Tris-HCI, pH 8.3; 500 mM KCI; 15 mM MgCl₂; 0.01 % (w/v) gelantin), 1 pmol template DNA, 100 pmol pUC-left, 100 pmol blocked pUC-right (3'-NH₂ modification), 200 µM dNTPs, any of the above mentioned dUTP concentrations, 2 U Taq DNA polymerase (e.g. Applied Biosystems) ad 100 µl aqua dest., and using the following cycler protocol: 1' 94 °C; 30 cycles consisting of 1' 94 °C, 1' 50 °C, 1.5' 72 °C. Purify the PCR products, e.g. with QiaQuick® (Qiagen) and pool the PCR-products as marker strands.
   Make an asymmetric PCR with the mixed PCR-products as template (antisense strand) by mixing 10 µl 10X PCR buffer (100 mM Tris-HCI, pH 8.3; 500 mM KCI; 15 mM MgCl₂; 0.01 % (w/v) gelantin), 1 pmol template DNA, 100 pmol blocked pUC-left (3'-NH₂ modification), 100 pmol pUC-right, 200 µM dNTPs, 2 U Taq DNA polymerase (e.g. Applied Biosystems) ad 100 µl aqua dest., and using the following cycler protocol: 1' 94 °C; 30 cycles consisting of 1' 94 °C, 1' 50 °C, 1.5' 72 °C. Purify the PCR products, e.g. with QiaQuick® (Qiagen) and pool the PCR-products as template strands.
6. Anneal 2 pmol of sense strand (with incorporated dU's) and 2 pmol of antisense strand in 100 mM NaCl (2' 95 °C, 95 °C -> 50 °C with 0,04 °C/s). Purify the annealed double stranded DNA, e.g. with QiaQuick®-Kit
7. Incubate 2 pmol of the annealed double stranded DNA with 1 U UDG (e.g. NEB) and 2 U Endonuclease IV (e.g. Epicentre) 1h at 37 °C in 20 µl UDG-Puffer (20 mM Tris-HCI, pH 8.0; 1 mM EDTA; 1 mM DTT). Add 80 µl of Vent-buffer (20 mM Tris-HCI, pH 8.8; 10 mM KCI; 10 mM (NH₄)₂SO₄; 2 mM MgSO₄; 0.1 % (v/v) Triton® X-100), 200 µM dNTPs and 2 U Vent(exo-) DNA polymerase (NEB). Incubate 5 min at 72 °C. Purify the DNA with QiaQuick® (Qiagen).
8. Incubate the product and 1 µg of pUC18 vector each with 1 U *Eco* RI (e.g. NEB) and 1 U *Hind* III (e.g. NEB) in Eco RI reaction buffer (100 mM Tris-HCl, pH 7.5; 50 mM NaCl; 10 mM MgCl₂; 0.025 % (v/v) Triton® X-100) for 2 h at 37 °C. Heat inactivate the enzymes for 20 min at 65 °C. Purify the cleavage products e.g. with QiaQuick®-Kit. ).
9. Ligate the product into the pUC18 vector using: 200 fmol vector, 600 fmol insert, 1 µl of 10X Ligation Buffer (500 mM Tris-HCI, pH 7.5; 100 mM MgCl₂; 100 mM DTT; 10 mM ATP, 250 µg/ml BSA), 5 Weiss Unit of T4 DNA ligase (e.g. NEB) ad 10 µl aqua dest. Incubate 1 h at room temperature and heat inactivate the enzyme for 10 min at 65 °C. Transform *E. coli* XL1-Blue with the ligated vector.

Protocol B: Generating more than one recombination event per gene For steps 1. to 4. see Protocol A.
5. Make an asymmetric PCR with the mixed PCR-products as template by mixing 10 µl 10X PCR buffer (100 mM Tris-HCI, pH 8.3; 500 mM KCI; 15 mM MgCl₂; 0.01 % (w/v) gelantin), 1 pmol template DNA, 100 pmol pUC-left, 100 pmol locked pUC-right (3'-NH₂ modification), 200 µM dNTPs, 2 µM dUTP, 2 U Taq DNA polymerase (e.g. Applied Biosystems) ad 100 µl aqua dest., and using the following cycler protocol: 1' 94 °C; 30 cycles consisting of 1' 94 °C, 1' 50 °C, 1.5' 72 °C. Purify the PCR products, e.g. with QiaQuick® (Qiagen) as marker strands.
   Make an asymmetric PCR with the mixed PCR-products as template by mixing 10 µl 10X PCR buffer (100 mM Tris-HCl, pH 8.3; 500 mM KCI; 15 mM MgCl₂; 0.01 % (w/v) gelantin), 1 pmol template DNA, 100 pmol blocked pUC-left (3'-NH₂ modification), 100 pmol pUC-right, 200 µM dNTPs, 2 U Taq DNA polymerase (e.g. Applied Biosystems) ad 100 µl aqua dest., and using the following cycler protocol: 1' 94 °C; 30 cycles consisting of 1' 94 °C, 1' 50 °C, 1.5' 72 °C. Purify the PCR products, e.g. with QiaQuick® (Qiagen) as template strands.
6. Anneal 2 pmol of marker strand (with incorporated dU's) and 2 pmol of template strand in 100 mM NaCl (2' 95 °C, 95 °C -> 50 °C with 0,04 °C/s). Purify the annealed double stranded DNA, e.g. with QiaQuick®-Kit (Qiagen).
7. Incubate 2 pmol of the annealed double stranded DNA with 1 U UDG (e.g. NEB) and 2 U Endonuclease IV (e.g. Epicentre) 1h at 37 °C in 20 µl UDG-Puffer (20 mM Tris-HCI, pH 8.0; 1 mM EDTA; 1 mM DTT). Add 2 U of UGI (Uracil Glycosylase Inhibitor, e.g. NEB). Add 80 µl of Vent-buffer (20 mM Tris-HCI, pH 8.8; 10 mM KCI; 10 mM (NH₄)₂SO₄; 2 mM MgSO₄; 0.1 % (v/v) Triton® X-100), 200 µM dNTPs, 2 µM dUTP and 2 U Vent(exo-) DNA polymerase (NEB). Incubate 5 min at 72 °C. Purify the DNA (e.g. with QiaQuick ®).
8. Reaneal the various strands in 100 mM NaCl (2' 95 °C, 95 °C -> 50 °C with 0,04 °C/s).
9. Repeat steps 7 and 8 several times (the number of cycles should equal the length of gene in bp / 100).
10. Incubate the product and 1 µg of pUC18 vector each with 1 U *Eco* RI (e.g. NEB) and 1 U *Hind* III (e.g. NEB) in *Eco* RI reaction buffer (100 mM Tris-HCI, pH 7.5; 50 mM NaCl; 10 mM MgCl₂; 0.025 % (v/v) Triton® X-100) for 2 h at 37 °C. Heat inactivate the enzymes for 20 min at 65 °C. Purify the cleavage products e.g. with QiaQuick®-Kit.
11. Ligate the product into the pUC18 vector using: 200 fmol vector, 600 fmol insert, 1 µl of 10X Ligation Buffer (500 mM Tris-HCI, pH 7.5; 100 mM MgCl₂; 100 mM DTT; 10 mM ATP, 250 µg/ml BSA), 5 Weiss Unit of T4 DNA ligase (e.g. NEB) ad 10 µl aqua dest. Incubate 1 h at room temperature and heat inactivate the enzyme for 10 min at 65 °C. Transform *E. coli* XL1-Blue with the ligated vector.

## Claims

1. A method for the production of polynucleotide molecules with modified properties, comprising the following steps:
(1) providing a population of source nucleic acid molecules, the individual nucleic acid molecules of said population having homologous and heterologous segments and having at least one marker nucleotide incorporated within its nucleic acid sequence;
(2) forming double-stranded polynucleotide molecules of the population of source nucleic acid molecules provided according to step (1) comprising double strands with heterologous segments (heteroduplices);
(3) producing single-stranded breaks at the incorporated marker nucleotides of the double-stranded heteroduplices produced according to step (2); and
(4) performing template-directed single-strand synthesis, with or without incorporation of marker nucleotides starting from single-stranded breaks produced according to step (3).

2. The method of claim 1, wherein
(i) more than one cycle, preferably at least two cycles, more preferably at least ten and most preferably at least twenty cycles, comprising the aforementioned steps (2) to (4) are performed; and/or
(ii) in all cycles but the last, step (4) is carried out with the incorporation of new marker nucleotides; and/or
(iii) steps (3) and (4) are carried out subsequently or contemporaneously.

3. The method of claim 1 or 2, wherein
(i) homologous segments have a length of at least 5, preferably of at least 10 and more preferably of at least 20 nucleotides and/or are not longer than 5,000 nucleotides, preferably not longer than 2,000 nucleotides, more preferably not longer than 1,000 nucleotides; and/or
(ii) the homologous segments are flanked by heterologous segments.

4. The method of any one of claims 1 to 3, wherein
(i) the incorporation of marker nucleotides into the nucleic acid molecules according to step (1) is achieved by using a template-directed polymerase reaction or by chemical synthesis of oligonucleotides; and/or
(ii) the production of double-stranded heteroduplex polynucleotides according to step (2) is achieved by hybridization of the homologous segments of complementary polynucleotides; and/or
(iii) the single-stranded breaks at the positions of the incorporated marker nucleotides of step (3) are nicks or gaps which are achieved by using enzymatic reactions; and/or
(iv) the template-directed single-strand synthesis of step (4) utilizes a polymerase.

5. The method of claim 1 or 4, wherein more than one cycle comprising steps (2) to (4) is performed and the average distance between the starting points of the template-directed synthesis according to step (4) in each of two consecutive cycles is controlled by adjusting the probability of incorporating marker nucleotides in step (4) of the first of the two consecutive cycles.

6. The method according to claim 5, wherein the probability of incorporating marker nucleotides is controlled by adjusting the ratio of concentrations of marker nucleotides to standard nucleotides; and/or wherein the probability of incorporating marker nucleotides is preferably lower than one and higher than the reciprocal of the source nucleic acid length in base pairs; and/or wherein the probability of incorporating marker nucleotides is altered from cycle to cycle.

7. The method of any one of claims 4 to 6, wherein the nucleic acid molecules are DNA molecules and in the template-directed polymerase reaction deoxyuridine triphosphate (dUTP) is utilized as a marker nucleotide in combination with the four standard deoxynucleoside triphosphates; and/or the uracil base of the incorporated marker uridine residues is separated from the ribose using an uracil-DNA glycosylase.

8. The method of any one of claims 4 to 6, wherein the nucleic acid molecules are DNA molecules and in the template directed polymerase reaction 8-oxo-doxyguanosine triphosphate (8-oxo-dGTP) is utilized as a marker nucleotide in combination with the four standard deoxynucleoside triphosphates; and/or the 8-oxo-guanine base of the incorporated 8-oxo-GMP residues is separated from the ribose using formamidopyrimidine-DNA glycosylases.

9. The method of any one of claims 4 to 6, wherein the nucleic acid molecules are DNA molecules and in the template directed polymerase reaction marker nucleotides with the following modified bases are used in combination with the four standard dNTPs: 3-methyladenine, 7-methyladenine, 3-methylguanine, 7-methylguanine, 7-hydroxyethylguanine, 7-chloroethylguanine, O2-alkylthymine, 02-alkylcytosine, 5-fluorouracil, 2,5-amino-5-formamidopyrimidine, 4,6-diamino-5-formamidopyrimidine, 2,6-diamino-4-hydroxy-5-formamidopyrimidine, 5-hydroxycytosine, 5,6-dihydrothymine, 5-hydroxy-5,6-dihydrothymine, thymine glycol, uracil glycol, isodialuric acid, alloxan, 5,6-dihydrouracil, 5-hydroxy-5,6-dihydrouracil, 5-hydroxyuracil, 5-formyluracil, 5-hydroxymethyluracil, hypoxanthine, 1,N6-ethenoadenine or 3,N4-ethenocytosine; and/or a DNA N-glycosylase which detects one of the aforementioned modified base, preferably *E.coli* endonuclease III or alkylbase DNA glycosylase, is utilized.

10. The method of any one of claims 4 to 6, wherein the nucleic acid molecules are DNA molecules and in the template directed polymerase reaction one, two, three or all four ribonucleoside triphosphates (rNTPs) are utilized as marker nucleotides in combination with the four standard dNTPs in the template directed polymerase reaction; and/or, the rNMP residues incorporated in the DNA polynucleotide are recognized by a specific ribonuclease H, preferably by human RNase H1.

11. The method of any one of claims 4 to 6, wherein the nucleic acid molecules are DNA molecules, any or all of the four ribonucleoside monophosphates (rNMPs) are used as marker nucleotides, and the marker strand is cleaved by alkaline hydrolysis at the rNMP residues, and/or the 2'- or 3'-rNMP at the 3'-end of a nick resulting from the alkaline hydrolysis is removed by a class II AP endonuclease, preferably by Exonuclease III or Endonuclease IV.

12. The method of any one of claims 4 to 11, wherein in step (4) the 3'OH-group at a nick or gap resulting from the enzymatic reactions is extended with a template directed polymerase reaction with or without the incorporation of additional marker nucleotides, preferably
(i) strands containing 5'-dRp group resulting from the action of a class II AP endonuclease are bound with a surplus of the corresponding template strands and the 3'-group is extended with a template directed polymerase; and/or
(ii) the 3'OH-group of the nick is template directed extended with a polymerase showing strong strand displacement properties; and/or
(iii) the 3'OH-group of the nick or gap is extended by a template directed polymerase showing a 5'3'-exonuclease activity or with other template directed polymerases in combination with an additional 5'3'-exonuclease.

13. The method of any one of claims 1 to 6, wherein the template strands in step (4) at which the template-directed single-strand synthesis takes place are RNA molecules, whereby an RNA-dependent DNA polymerase, preferably AMV reverse transcriptase from the avian myeloblastosis virus, HIV reverse transcriptase from the human immunodeficiency virus or MMLV reverse transcriptase from the Moloney murine leukemia virus are used for the template-directed single-strand synthesis.

14. A kit for carrying out the method as defined in any one of claims 1 to 13, preferably said kit containing at least one of the following components:
(i) buffer for production of double-stranded polynucleotides;
(ii) marker nucleotides for incorporation in the polynucleotide molecules;
(iii) agents permitting the single-stranded breaks at the incorporated marker nucleotides;
(iv) buffers for carrying out the incorporation of the marker nucleotides and producing the single-stranded breaks at these sites;
(v) agents permitting the template-directed polymerization of a polynucleotide strand starting from the single-stranded break; and
(vi) buffer for carrying out this polymerization reaction.
